# EUROPEAN PATENT APPLICATION

(11) **EP 4 394 024 A1**
(43) Date of publication of application: **03.07.2024**
(21) Application number: 22383302.1
(22) Date of filing: 28.12.2022
(51) Int. Cl.: C12M 1/00, B01D 53/84, C12M 3/00, C12M 1/06

(54) **MODULAR AND SCALABLE BIOLOGICAL PLANT FOR NOX ABATEMENT**

(71) Applicant: Bromalgae, S.L., 48901 Barakaldo (Vizcaya) (ES)
(72) Inventor: Garcia Bárcena, Teresa, 48901 Bizkaia (ES); Alonso Hornes, Julen, 48901 Bizkaia (ES); Barredo Gallarza, Guillermo, 48901 Bizkaia (ES)
(74) Representative: Balder IP Law, S.L.

(57) **Abstract**

The present invention is a modular and vertically scalable biological plant for NOx abatement. The plant comprises: a support structure formed by a plurality of vertically superimposed modules, a plurality of photobioreactor tanks suitable for carrying out a microalgae culture inside, wherein each tank is arranged in one of the modules of the structure, such that all the tanks are vertically aligned with respect to the floor. The plant further comprises: means of temperature regulation inside each photobioreactor tank, means of culture fluid agitation inside each of the photobioreactor tanks, and means of culture lighting inside each of the tanks. The plant requires a minimum footprint so that it can be installed in industrial plants already in operation.

## Description

### TECHNICAL FIELD

The present invention relates in general to a biological plant for NOx abatement.

An object of the invention is to provide a modular and scalable biological plant that requires a minimum footprint so that it can be installed in industrial plants already in operation, where it is possible that there is insufficient space for the plant to grow above ground.

### STATE OF THE ART

NOx are the most abundant pollutants in the air. They are mainly anthropogenic and originate from combustion processes. The accumulation of NOx in the atmosphere causes serious environmental damage, such as the acidification of ecosystems or metabolic damage, but also on human health, generating respiratory and other system affections.

Currently, chemical methods are the most widely used for the reduction of environmental NOx, but such processes are not only costly, but can also generate secondary pollutants. Therefore, biological processes are presented as an alternative to this problem, reducing energy and economic costs and eliminating the generation of secondary pollutants.

Thus, microalgae are a good option for the treatment of NOx, as they can reduce emissions by using it as a nutrient. In addition, obtaining microalgal biomass makes it possible to obtain high added value compounds such as pigments, proteins or oils.

### DESCRIPTION OF THE INVENTION

The invention relates to a modular and scalable biological plant for NOx abatement, comprising a support structure formed by a plurality of vertically superimposed modules such that each module defines a floor of the structure.

The plant further has a plurality of photobioreactor tanks suitable for containing a culture fluid, and for carrying out a microalgae culture therein.

The function of the photobioreactor tank (PBR) is to facilitate and maintain the cultivation of microalgae inside it, forming a compact, closed and automatic cultivation system. It is a complex piece of equipment that includes all the elements and subsystems necessary to carry out the physical and biological functions, as an integrated element in the NOx abatement plant.

Each tank is arranged in one of the modules of the structure, i.e. on one of the floors of the structure, so that all the tanks are aligned vertically with respect to the ground, i.e. one above the other.

The plant further comprises ducts for the inlet of the gas to be abated individually to each of the tanks and for the outlet of the abated gas from the tanks, and ducts connected to the photobioreactor tanks for the individual inlet and outlet of the culture fluid from the tanks. Preferably, the gas inlet to each tank is carried out through means for the dispersion of the gas by bubbling within the fluid.

For this function, at the bottom of each tank there is a distributor of tubes and nozzles for bubbling the plant's treatment gases through the tank, so that the gas, driven by a system of blowers, diffuses the gases to be treated together with other nutrients supplied to the system, into the culture fluid, in such a way that a uniform distribution of gases is achieved throughout the tank.

The plant further comprises: means of temperature regulation inside each photobioreactor tank, means of agitation of the culture fluid inside each of the photobioreactor tanks, and means of illumination of the culture inside each of the tanks.

The temperature regulation means may include a heat exchanger inside each tank, and are fed by a heat transfer fluid to perform the function of tempering and maintaining the temperature of the fluid within the margins necessary for cultivation. The heat transfer fluid is distributed to each of the tanks by means of the corresponding network of pipes.

The tank has an agitation and fluid movement system designed with a triple objective: (i) to enable the distribution of microalgae inside the tank; (ii) to favour the distribution of gases and nutrients throughout the volume of fluid, and given the sensitivity of the microalgae to exposure to light, and taking into account the possible, albeit slight, differences in light intensity at different points in the volume of liquid; (iii) to promote movement and change of position inside the tank to achieve uniform doses of light intensity in the microalgae.

To facilitate the biological processes inside the tank and to have the necessary lighting for the photosynthesis of the microalgae, there is an artificial LED lighting system, capable of generating and adequately distributing the light to the entire volume of the tank.

Preferably, each photobioreactor tank has a cylindrical configuration, and is closed by a conical or inclined lower base and a fluid outlet in the lower part of the lower base, for the exit by gravity of the microalgae culture that settles to the bottom. The position of the culture outlet, together with the sloping design of the bottom of the tank, enables the drainage of the culture towards the corresponding filtering system, and the preparation of the culture, as well as the treatment and replenishment of the water in the system.

The tank is preferably closed at the top by a conical or inclined top base. The treatment gases are discharged into the chimney system via the corresponding pipes (fluid lines) in the upper base of the tank, which are driven by the corresponding blowers, also within the gas system.

Preferably, each photobioreactor tank has a heat radiator attached to the external surface of the tank, either on the side surface and/or on the upper base, to dissipate heat from the illumination means.

As can be seen, each photobioreactor tank integrates most of the components necessary for the cultivation of microalgae, namely:
- fluid feed system to the tank; process water, suitably treated to create the culture fluid for the microalgae fauna;
- inlet, distribution and bubbling system for plant treatment gases, coming from the washing tower of the gas system. Other nutrients needed in the process will also be introduced through this system;
- venting and venting system for the treatment gases, for their collection and conduction to the plant stack, also within the plant gas system;
- agitation system, to facilitate the correct distribution of nutrients throughout the tank volume, as well as to carry out the appropriate movement of the microalgae in such a way as to achieve a uniform distribution of lighting throughout the culture;
- LED lighting system inside the tank, in order to achieve a correct light distribution for the development of the culture and photosynthesis reactions in the tank;
- heat exchangers of the tank temperature control system by means of heat transfer fluid, to achieve and maintain the set temperature of the culture fluid.

Together with the other auxiliary systems of the plant (gases, heat transfer fluid, cultivation...) the PBR tank system works automatically to facilitate, maintain and cultivate the microalgae in the NOx abatement plant in which it is integrated. The PBR tank is filled with process water by means of the correspondingly arranged pipes (fluid conduits). Likewise, the necessary water is replenished through these elements after each crop emptying.

Preferably, each tank has a cylindrical body coaxial with the body of the tank, so that the fluid agitation means are arranged inside the cylinder. The cylindrical body has a double wall to integrate the means of illumination into the wall.

With the system in operation, and with the appropriate regulation of the process parameters (lighting, gas flow, nutrients, agitation, cultivation, etc.), a continuous system of treatment and abatement of NOx gases is achieved, as well as the cultivation of a high added value algal biomass after processing.

The plant of the invention allows the standardisation of components and subsystems, facilitating the adaptation of the scale to the needs of each process, by repeating the structural base module or applying the corresponding scaling factors.

The distribution of the tanks aligned vertically and supported by the modular structure means that the space required for its installation is very small, so it can be installed in existing industrial plants with space limitations.

### BRIEF DESCRIPTION OF THE DRAWINGS

To complete the description and in order to provide a better understanding of the invention, a set of drawings is provided. These drawings form an integral part of the description and illustrate embodiments of the invention, which should not be interpreted as restricting the scope of the invention, but just as examples of how the invention can be carried out. The drawings comprise the following figures:
Figure 1.- shows a front elevational view of a plant according to the invention, formed by six floors with six photobioreactor tanks.
Figure 2.- shows three views of the support structure itself, a perspective view (Figure A), an elevational view (Figure B) and a top plan view (Figure C).
Figure 3.- shows an elevation and section view of one of the photobioreactor tanks.
Figure 4.- shows a plan and section view of one of the photobioreactor tanks.
Figure 5.- shows an elevation view of the plant with the gas circuit.
Figure 6.- shows a flow diagram of the gas feed and outlet process.

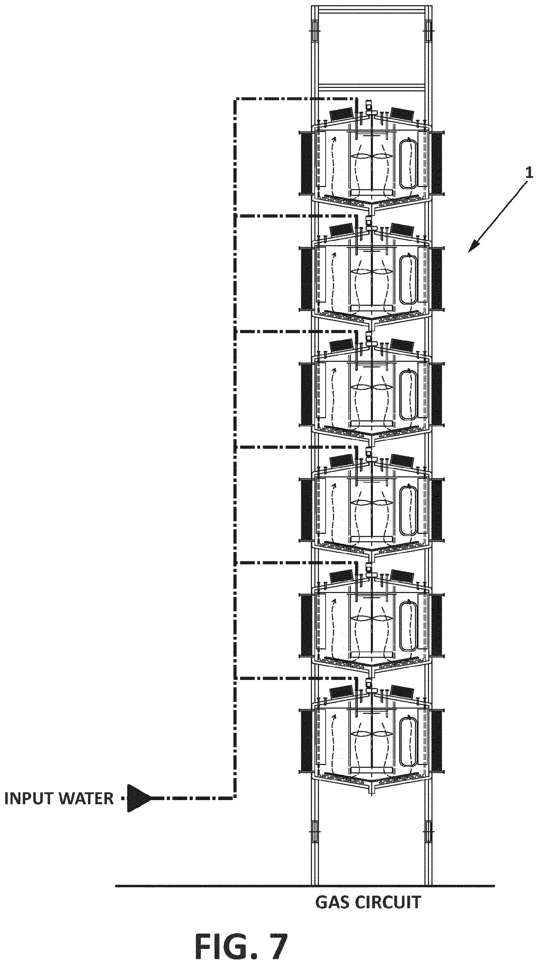

Figure 7.- shows an elevation view of the plant with the fluid (water) feed circuit.

### DESCRIPTION OF WAYS OF CARRYING OUT THE INVENTION

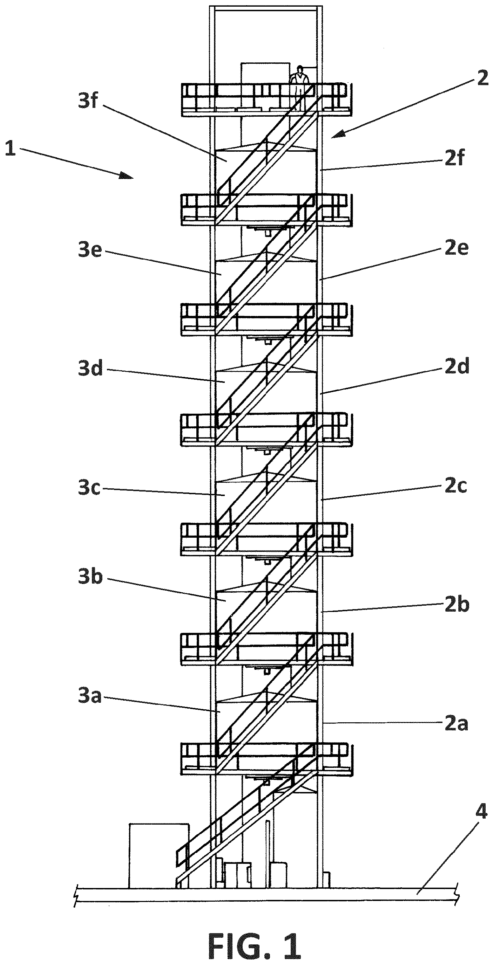

**Figure 1** shows an example of the NOx abatement plant (1), comprising a support structure (2) formed by a plurality of modules (2a - 2f) vertically superimposed, so that each module defines a floor of the structure.

In each module (2a - 2f) of the structure (2) there is a photobioreactor tank (3a - 3f) suitable for carrying out a microalgae culture inside, and suitable for containing a culture fluid, such that all tanks are vertically aligned with respect to the floor (4).

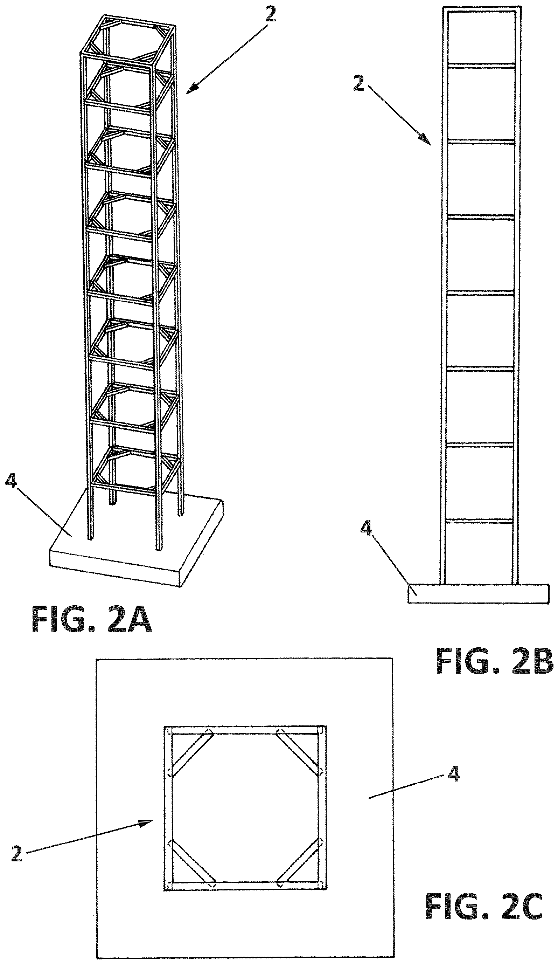

As shown in **Figure 2****,** the structure (2) is vertical and consists of cubic modules that are assembled one on top of the other.

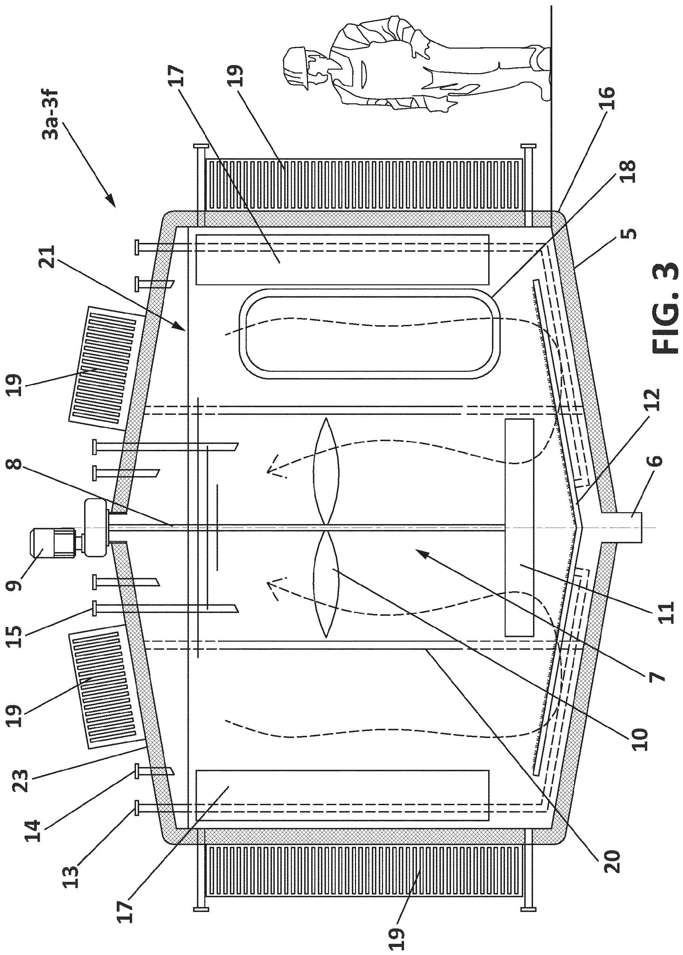

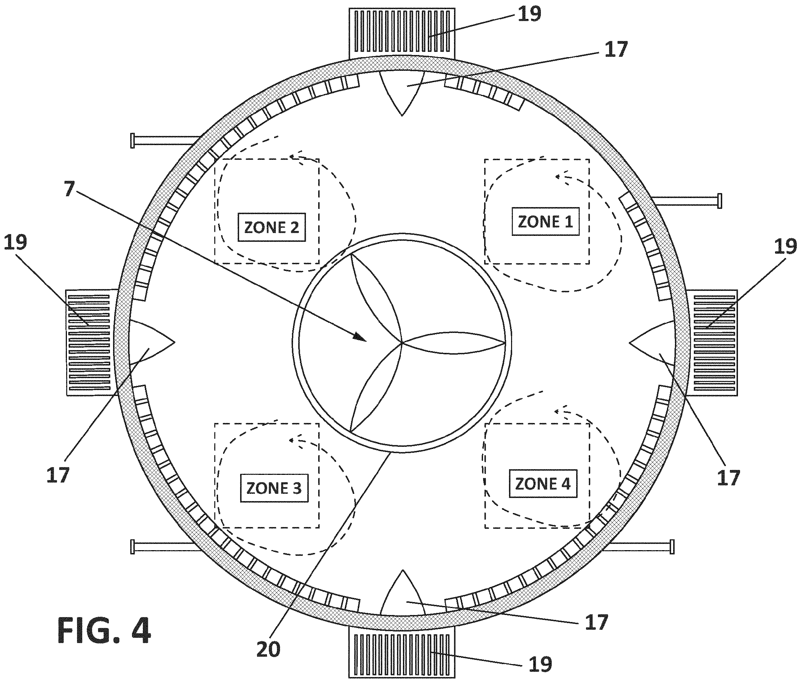

**Figures 3** and **4** show an elevation and section view of one of the photobioreactor tanks (3a - 3f), which as can be seen has a cylindrical configuration in its main body, and is closed by a conical lower base (5) and a fluid outlet (6) in the lower part of the lower base, for the outlet by gravity of the microalgae culture that settles towards the bottom of the tank. At the upper level, the tank is closed by a conical upper base (23).

All tanks (3a - 3f) of the plant (1) are arranged coaxially when mounted on the structure (2).

The entire outer surface of each tank (3a - 3f) can be covered with a thermal insulating material (16), such as rock wool. A manhole (18) allows an operator to enter the interior of the tank to carry out maintenance work.

Each of the tanks (3a - 3f) incorporates culture fluid agitation means (7). These means (7) comprise a rotating shaft (8) driven by a motor (9), first blades (10) at mid-height of the tank attached to the shaft, and second blades (11) attached to the shaft and at the lower level of the tank.

In addition, each of the tanks (3a - 3f) incorporates means for the entry of gas by bubbling, consisting of perforated ducts (12) located at the lower level of the tank, which receive the inlet gas through gas inlet ducts (13), which emerge through the upper base (23) of the tank towards the outside.

In addition, each of the tanks (3a - 3f) incorporates a heat exchanger in the gas inlet ducts (13) and in the gas outlet ducts (14) to regulate the temperature inside each photobioreactor tank. The heat exchanger is sized according to the volume of fluid to be heated or cooled, as well as the maximum/minimum operating temperatures, in order to avoid unacceptable temperature gradients inside the tank that could endanger the culture.

This is an auxiliary system of the plant whose function is to provide thermal energy to the PBR tanks, so that it can adapt the temperature of the fluid to the operating temperatures of the system, keeping it within the stipulated operating margins. The heat transfer fluid temperature control system is therefore capable of raising or lowering the temperature of each PBR culture tank, as well as overcoming the thermal inertia that may occur at any given time, without altering the biological functioning of the plant. The system uses water as an exchange fluid and has equipment for both hot water production and cooling. Equipment and systems for pumping the fluid, heat exchangers in each PBR tank, as well as devices for regulating and controlling the entire system.

The system operates in a closed circuit, taking water from the mains for filling. By means of the appropriate equipment, it allows both heating and cooling of the heat transfer fluid. Equipment is available for hot water production. The equipment will be able to raise the temperature of the water to the set point temperature of the systems and exchange circuits. The equipment is designed as a stand-alone unit, consisting of a dedicated boiler. However, it can be an equipment that uses residual or free energies, such as heat recovery from industrial processes or solar thermal energy.

The culture fluid, preferably water, enters through a conduit (15) in the upper base (23).

In addition, each of the tanks (3a - 3f) incorporates a means of culture lighting within each of the tanks. These lighting means may consist of baffles (17) fitted with LEDs. Radiators or heat sinks (19) are arranged on the outer side surface of the tank, and on the upper base, to dissipate the heat generated by the lighting means.

Each of the tanks (3a - 3f) has in its interior a cylindrical body (20) coaxial with the body of the tank, so that inside the cylinder the fluid agitation means (7) are arranged, so that a fluid circulation (21) is generated inside the tank as indicated by the arrows and dotted lines in **Figures 3** and **4****.**

Furthermore, this cylindrical body (20) is double-walled and integrates in the double wall means of illumination.

As can be seen, all the elements that form part of the tanks (3a - 3f) are installed together with the tank, either inside the tank or attached to its external enclosure. All the equipment and auxiliary elements of the tank are arranged and assembled together with it, facilitating the manufacturing and installation works.

Each tank is designed to treat the volume of plant that proportionally corresponds to it. It also has the appropriate volume for this purpose. In the example of the figures, with 6 tanks of 38 m3 each, the total volume of fluid to be conditioned will be about 230 m3, capable of treating 2000 Nm3/h of combustion gases. The design of each tank takes into account its design parameters, in particular the temperature, with the appropriate design of its heating and heat transfer fluid exchange system.

As an example, the tank dimensions can be as follows: inner diameter (D1): 4154 mm; height to liquid film (H1): 2851 mm; tank volume: 38 m3; bottom slope: 17 %; construction material: stainless steel.

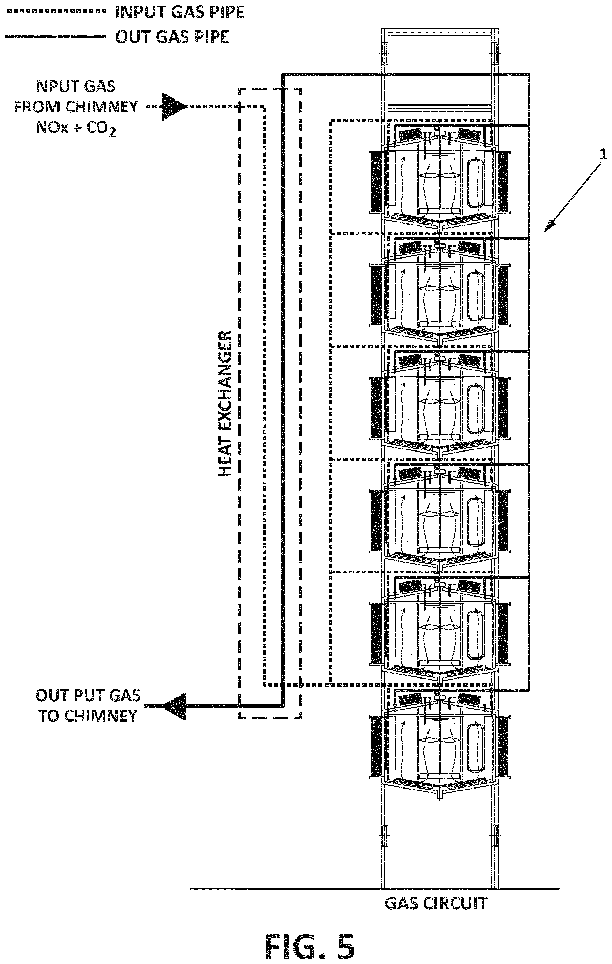

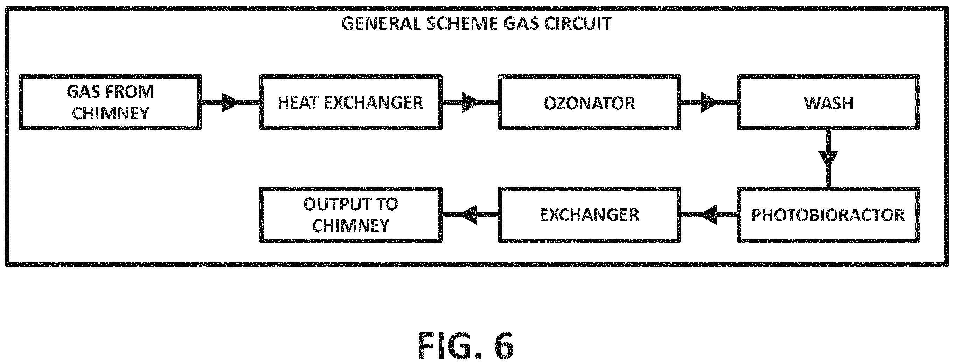

**Figure 5** shows the gas circuit in the plant (1), and **Figure 6** shows a flow diagram of the gas inlet and outlet process. The input gases to be abated are the emission gases from a factory chimney. Using appropriate blower systems, the gases are passed through an external heat exchanger (22), where the temperature of the gases is reduced to the setpoint values.

An optional air cooler is available for exceptional situations. The gases are then passed through an ozonisation system where the NOx are oxidised to NO3 and subsequently scrubbed in a corresponding gas scrubbing tower. These nitrogen compounds serve as nutrients for the algae grown in the PBR tanks. Once scrubbed, the gases are ready to be bubbled into the photobioreactor tanks (PBR), where they serve as nutrients for the algae.

Once expelled from the tanks through the corresponding venting systems, the gases are propelled, by means of the corresponding blower systems, through the heat exchanger, where they raise their temperature at the expense of reducing the temperature of the treatment gases of the new cycle; they are then expelled into the atmosphere through the plant's chimney.

The gas flow passes through the heat exchanger, where its temperature will be reduced to meet the needs of the rest of the system. The exchange fluid will be the return gases to the chimney, after passing through the treatment plant. With this design of the heat exchanger, two objectives are achieved: On the one hand, the temperature of the supply and inlet gases to the abatement plant is reduced. At the same time, the flue gas will be heated to facilitate its emission and dispersion in the atmosphere once it is returned to the chimney.

This gas supply circuit includes all the necessary equipment and regulation elements to control the appropriate flow rates to each of the PBR tanks. Likewise, the system enables the operation of the system as a whole or independently in each tank, acting on the bypass devices when necessary.

All the equipment is arranged at the foot of the abatement tower, forming modular "Skid" type assemblies, which facilitate their manufacture, assembly and possible relocation to another plant, contributing to the modular nature of the plant.

## Claims

1. Modular and scalable biological plant for NOx abatement, comprising:
a support structure formed by a plurality of vertically superimposed modules, each module defining a floor of the structure,
a plurality of photobioreactor tanks, each suitable for conducting a microalgae culture therein,
and suitable for containing a culture fluid,
wherein each tank is arranged in one of the modules of the structure, such that all tanks are vertically aligned with respect to the ground,
ducts for the inlet of the gas to be depressurised individually to each of the tanks and for the outlet of the depressurised gas from the tanks,
ducts connected to the photobioreactor tanks for the individual inlet and outlet of fluid from the tanks,
means of temperature regulation inside each photobioreactor tank, and
means of illumination of the culture inside each of the tanks.

2. Plant according to claim 1, wherein each of the tanks incorporates means of agitation of the culture fluid.

3. Plant according to claim 1 or 2, wherein each of the tanks incorporates means for the entry of gas by bubbling.

4. Plant according to any of the previous claims, incorporating a heat exchanger in the gas inlet and outlet ducts.

5. Plant according to any of the previous claims, where each photobioreactor tank has a cylindrical configuration, closed by a conical or inclined lower base and a fluid outlet in the lower part of the lower base, for the outlet by gravity of the microalgae culture that settles to the bottom.

6. Plant according to claim 5, where the tank is closed by a conical or inclined upper base.

7. Plant according to any of the previous claims, where each photobioreactor tank has a heat radiator attached to the external surface of the tank, to evacuate heat from the means of illumination.

8. Plant according to any of the previous claims, where the means of temperature regulation include a heat exchanger in each of the tanks.

9. Plant according to any of the previous claims, where each photobioreactor tank has in its interior a cylindrical body coaxial with the body of the tank, so that in the interior of the cylinder the means of fluid agitation are arranged, and because the cylindrical body has a double wall and integrates in the double wall some means of illumination.
